# EUROPÄISCHE PATENTSCHRIFT

(11) **EP 1 351 765 B1**
(45) Veröffentlichungstag und Bekanntmachung des Hinweises auf die Patenterteilung: **05.01.2005**
(21) Anmeldenummer: 00991257.7
(22) Anmeldetag: 22.12.2000
(51) Int. Cl.: B01J 35/10, B01J 21/06, B01J 23/02, B01J 23/08, B01J 23/70, B01J 37/00, C07C 5/32

(54) **KATALYSATOR MIT BIMODALER PORENRADIENVERTEILUNG**
CATALYST WITH BIMODAL PORE RADIUS DISTRIBUTION
CATALYSEUR A REPARTITION BIMODALE DU RAYON DES PORES

(43) Veröffentlichungstag der Anmeldung: 15.10.2003
(73) Patentinhaber: BASF AKTIENGESELLSCHAFT, 67056 Ludwigshafen (DE)
(72) Erfinder: HEINEKE, Daniel, The Woodlands, TX 77381 (US); HARTH, Klaus, 67317 Altleiningen (DE); STABEL, Uwe, 67166 Otterstadt (DE)
(86) Internationale Anmeldenummer: PCT/EP2000/013160
(87) Internationale Veröffentlichungsnummer: WO 2002/051547

(56) Entgegenhaltungen:
- EP-A- 0 400 306
- EP-A- 0 421 077
- EP-A- 0 761 307
- EP-A- 0 803 488
- EP-A- 1 074 299
- EP-A- 1 074 301
- US-A- 3 898 155
- US-A- 3 960 710
- US-A- 4 102 822
- US-A- 4 454 026
- US-A- 4 549 957
- US-A- 4 568 655
- US-A- 5 221 656
- US-A- 5 322 821

## Beschreibung

Die vorliegende Erfindung betrifft Katalysatoren mit bimodaler Porenradienverteilung, die a) Zirkondioxid und b) gegebenenfalls Aluminiumoxid, Titandioxid und/oder Siliciumoxid und c) mindestens ein Element der ersten oder zweiten Hauptgruppe, ein Element der dritten Nebengruppe, ein Element der achten Nebengruppe des Periodensystems der Elemente, Lanthan und/oder Zinn enthalten.

Aus der US-A-5,220,091 sind Katalysatoren bestehend aus Pt/Sn als Aktivkomponente auf einem Zn-Spinell-Träger zur Dehydrierung von kleinen Kohlenwasserstoffmolekülen wie Isobutan mit Wasserdampf als Verdünnungsmittel bekannt. Hinsichtlich ihrer Performance sind diese Katalysatoren verbesserungsbedürftig, denn es werden trotz hoher Verdünnung des Feed mit Wasserdampf (Verhältnis 4:1) bei hohen Reaktionstemperaturen von 600°C nur relativ geringe Umsätze und Selektivitäten erzielt. Ebenfalls verbesserungswürdig ist die Standzeit der Katalysatoren, denn es muß nach einer Betriebszeit von nur 7 h regeneriert werden.

Aus der US-A-4,788,371 sind Pt/Sn/Cs/Al₂O₃-Katalysatoren zur Dehydrierung von Kohlenwasserstoffen durch Wasserdampffahrweise (z.B. Wasserdampf/Propan 10:1) bekannt. Trotz des hohen Verdünnungsgrad werden nur geringe Umsätze von 21% erreicht.

Aus der WO-A-94/29021 sind Katalysatoren auf der Basis von Mischoxiden von Magnesium und Aluminium mit einem Edelmetall der Gruppe VIII, einem Metall der Gruppe IVa und gegebenenfalls einem Alkalimetall der Gruppe Ia des Periodensystems der Elemente zur Dehydrierung z.B. eines Gasgemisch aus H₂O/Propan/H₂/N₂ im Verhältnis 8:7:1:5 bekannt. Nachteilig für eine technische Anwendung dieser Katalysatoren ist ihre geringe Härte, die einen technischen Einsatz schwierig macht. Weiterhin sind diese Katalysatoren in ihrer Performance, insbesondere bei niedrigen Reaktionstemperaturen verbesserungsbedürftig. Ein weiterer Nachteil ist die aufwendige Fahrweise, die zur Erhaltung der Performance den Zusatz von Wasserstoff zum Feed und die Zumischung von Stickstoff zur weiteren Verdünnung erfordert.

EP-A-803 488 offenbart in situ hergestellte Rutheniumkatalysatoren auf metallischen oder auch oxidischen Trägern wie etwa Zirkondioxid, Aluminiumoxid und/oder Siliciumdioxid. Die Träger können eine bimodale Porenradienverteilung aufweisen und sind vorzugsweise makroporös. Die Katalysatoren können weiterhin Elemente der ersten, dritten oder achten Nebengruppe enthalten. Sie werden zur Hydrierung oder Dehydrierung von Kohlenwasserstoffen verwendet.

WO 98/29365 lehrt Dehydrierkatalysatoren mit einem dehydrieraktiven Element aus der 6. oder der 8. Nebengruppe des Periodensystems der Elemente, einem Rhenium- und/oder Zinn-Stabilisator sowie mindestens einem Promotor aus der Alkali- oder Erdalkaligruppe, der 3. Nebengruppe, der 3. Hauptgruppe und/oder Zink auf monoklinem Zirkondioxid.

Der vorliegenden Erfindung lag deshalb die Aufgabe zugrunde, den zuvor genannten Nachteilen abzuhelfen.

Demgemäß wurden neue und verbesserte Katalysatoren mit bimodaler Porenradienverteilung gefunden, bestehend im Wesentlichen aus
a) 10 bis 99,9 Gew.-% Zirkondioxid, das zu 50 bis 100 Gew.-% in monokliner Modifikation vorliegt, und
b) 0 bis 60 Gew.-% Aluminiumoxid, Siliciumdioxid und/oder Titandioxid und
c) 0,1 bis 10 Gew.-% mindestens eines aus der ersten oder zweiten Hauptgruppe, der dritten oder achten Nebengruppe des Periodensystems der Elemente und Zinn gewählten Elements,
mit der Maßgabe, daß die Summe der Gewichtsprozente 100 ergibt, ein Verfahren zur Dehydrierung von C₂- bis C₁₆-Kohlenwasserstoffen und die Verwendung dieser Katalysatoren dazu sowie ein Verfahren zur Herstellung dieser Katalysatoren.

Die erfindungsgemäßen Katalysatoren bestehen im Wesentlichen aus, und bevorzugterweise bestehen aus
a) 10 bis 99,9 Gew.-%, bevorzugt 20 bis 98 Gew.-%, besonders bevorzugt 30 bis 95 Gew.-% Zirkondioxid, das zu 50 bis 100 Gew.-%, bevorzugt zu 60 bis 99 Gew.-%, besonders bevorzugt zu 70 bis 98 Gew.-% in monokliner Modifikation vorliegt, und
b) 0 bis 60 Gew.-%, bevorzugt 0,1 bis 50 Gew.-%, besonders bevorzugt 1 bis 40 Gew.-%, insbesondere 5 bis 30 Gew.-% Aluminiumoxid, Siliciumdioxid und/oder Titandioxid in Form von Rutil oder Anatas, für Siliciumdioxid bevorzugt 0,5 bis 25 Gew.-%, und
c) 0,1 bis 10 Gew.-%, bevorzugt 0,2 bis 8 Gew.-%, besonders bevorzugt 0,5 bis 5 Gew.-%, mindestens eines aus der ersten oder zweiten Hauptgruppe, der dritten oder achten Nebengruppe des Periodensystems der Elemente und Zinn gewählten Elements,
wobei sich die Summe der Gewichtsprozente zu 100 ergibt.

Der Gehalt der erfindungsgemäßen Katalysatoren an einem Edelmetall beträgt in der Regel 0,01 bis 5 Gew.-%, bevorzugt 0,1 bis 1 Gew.-%, besonders bevorzugt 0,2 bis 0,5 Gew.-%.

Die erfindungsgemäßen Katalysatoren enthalten 70 bis 100 %, bevorzugt 75 bis 98 %, besonders bevorzugt 80 bis 95 % der Poren kleiner als 20 nm oder zwischen 40 und 5000 nm.

Zur Herstellung der erfindungsgemäßen Katalysatoren können Precursoren der Oxide des Zirkons, Titans, Siliciums und Aluminiums (bilden den Träger), die sich durch Calcinieren in die Oxide umwandeln lassen, eingesetzt werden. Diese können nach bekannten verfahren, zum Beispiel nach dem Sol-Gel-Verfahren, Fällung der Salze, Entwässern der entsprechenden Säuren, Trockenmischen, Aufschlämmen oder Sprühtrocknen hergestellt werden. Zum Beispiel kann zur Herstellung eines ZrO₂ • xAl₂O₃ • xSiO₂-Mischoxides zunächst ein wasserreiches Zirkonoxid der allgemeinen Formel ZrO₂ • xH₂O durch Fällung eines geeigneten Zirkon-haltigen Precursors hergestellt werden. Geeignete Precursoren des Zirkons sind zum Beispiel Zr(NO₃)₄, ZrOCl₂, oder ZrCl₄. Die Fällung selbst erfolgt durch Zugabe einer Base wie zum Beispiel NaOH, KOH, Na₂CO₃ und NH₃ und ist beispielsweise in der EP-A-849 224 beschrieben.

Zur Herstellung eines ZrO₂ • xSiO₂-Mischoxides kann der zuvor erhaltene Zr-Precursor mit einem Si-haltigen Precursor gemischt werden. Gut geeignete Precursoren des SiO₂ sind zum Beispiel wasserhaltige Sole des SiO₂ wie Ludox™. Die Mischung der beiden Komponenten kann beispielsweise durch einfaches mechanisches Vermischen oder durch Sprühtrocknen in einem Sprühturm erfolgen.

Bei der Verwendung von Mischoxiden besteht die Möglichkeit der gezielten Beeinflussung der Porenstruktur. Die Korngröße der verschiedenen Precursoren beeinflussen das Porengefüge. So lassen sich beispielsweise über die Verwendung von Al₂O₃ mit einem geringen Glühverlust und einer definierten Korngrößenzusammensetzung Makroporen im Gefüge erzeugen. Bewährt hat sich in diesem Zusammenhang die Verwendung von Puralox (Al₂O₃ mit einem Glühverlust von etwa 3%).

Zur Herstellung eines ZrO₂ • xSiO₂ • xAl₂O₃-Mischoxides kann die wie oben beschrieben erhaltene SiO₂ • xZrO₂-Pulvermischung mit einem Al-haltigen Precursor versetzt werden. Dies kann zum Beispiel durch einfaches mechanisches Mischen in einem Kneter erfolgen. Die Herstellung eines ZrO₂ • xSiO₂ • xAl₂O₃-Mischoxides kann aber auch in einem einzigen Schritt durch Trockenmischung der einzelnen Precursoren erfolgen.

Die Mischoxide haben gegenüber reinem ZrO₂ unter anderem den Vorteil, daß sie sich leicht verformen lassen. Dazu wird die erhaltene Pulvermischung im Kneter mit einer konzentrierten Säure versetzt und kann dann in einen Formkörper, z.B. mittels einer Strangpresse oder eines Extruders überführt werden.

Eine weitere Möglichkeit zur gezielten Herstellung der Träger mit speziellen Porenradienverteilungen für die erfindungsgemäßen Katalysatoren besteht in der Zugabe verschiedener Polymere während der Herstellung, die durch Calcinierung teilweise oder vollständig entfernt werden, wobei Poren in definierten Porenradienbereichen entstehen. Die Mischung der Polymere und der Oxid-Precursoren kann beispielsweise durch einfaches mechanisches Vermischen oder durch Sprühtrocknen in einem Sprühturm erfolgen.

Besonders bewährt zur Herstellung der Träger mit bimodaler Porenradienverteilung hat sich die Verwendung von PVP (Polyvinylpyrrolidon). Wird dieses in einem Herstellschritt zu einem oder mehreren Oxid-Precursoren aus der Reihe der Elemente Zr, Ti, Al oder Si gegeben, so entstehen nach dem Calcinieren Makroporen im Bereich von 200 bis 5000 nm. Ein weiterer Vorteil der Verwendung von PVP ist die leichtere Verformbarkeit des Trägers. So können aus frisch gefälltem wasserhaltigem ZrO₂ • xH₂O, das vorher bei 120°C getrocknet wurde unter Zusatz von PVP und Ameisensäure auch ohne weitere Oxid-Precursoren mühelos Stränge mit guten mechanischen Eigenschaften hergestellt werden.

Die Mischoxid-Träger der erfindungsgemäßen Katalysatoren weisen nach der Calcinierung im allgemeinen höhere BET-Oberflächen als reine ZrO₂-Träger auf. Die BET-Oberflächen der Mischoxid-Träger liegen im allgemeinen zwischen 40 und 300 m²/g, bevorzugt zwischen 50 und 200 m²/g, besonders bevorzugt zwischen 60 und 150 m²/g. Das Porenvolumen der erfindungsgemäßen Katalysatoren beträgt üblicherweise 0,1 bis 0,8 ml/g, bevorzugt 0,2 bis 0,6 ml/g. Der durch Hg-Porosimetrie bestimmbare mittlere Porendurchmesser der erfindungsgemäßen Katalysatoren liegt zwischen 5 und 20 nm, bevorzugt zwischen 8 und 18 nm. Weiterhin vorteilhaft ist ein Anteil an Poreri > 40 nm, der zwischen 10 und 80 % bezogen auf das Porenvolumen beträgt.

Die Calcinierung der Mischoxid-Träger erfolgt zweckmäßigerweise nach dem Aufbringen der Aktivkomponenten und wird bei Temperaturen von 400 bis 700°C, bevorzugt von 500 bis 650°C, besonders bevorzugt bei 560 bis 620°C durchgeführt. Die Calciniertemperatur sollte dabei üblicherweise mindestens so hoch sein wie die Reaktionstemperatur der Dehydrierung für die die erfindungsgemäßen Katalysatoren eingesetzt werden.

Charakteristisch für die erfindungsgemäßen Katalysatoren ist die bimodale Porenradienverteilung. Die Poren liegen dabei im Bereich bis 20 nm und zwischen 40 und 5000 nm. Bezogen auf das Porenvolumen machen diese Poren mindestens 70% der Poren aus. Der Anteil an Poren kleiner als 20 nm beträgt dabei im allgemeinen zwischen 20 und 60%, der Anteil an Poren zwischen 40 und 5000 nm beträgt im allgemeinen ebenfalls 20 bis 60 %.

Die Dotierung der Mischoxide mit einer basischen Verbindung kann entweder während der Herstellung, zum Beispiel durch gemeinsame Fällung oder nachträglich zum Beispiel durch Tränken des Mischoxides mit einer Alkali- oder Erdalkalimetallverbindung oder einer Verbindung der 3. Nebengruppe oder einer Seltenerdmetall-Verbindung erfolgen. Besonders geeignet zur Dotierung sind K, Cs und Lanthan.

Die Aufbringung der dehydrieraktiven Komponente, das üblicherweise ein Metall der VIII. Nebengruppe ist, erfolgt in der Regel durch Tränkung mit einem geeignetem Metallsalzprecursor, der sich durch Calcinieren in das entsprechende Metalloxid umwandeln läßt. Statt durch Tränkung kann die dehydrieraktive Komponente aber auch durch andere Verfahren wie beispielsweise Aufsprühen des Metallsalzprecursors erfolgen. Geeignete Metallsalzprecursoren sind z.B. die Nitrate, Acetate und Chloride der entsprechenden Metalle, möglich sind auch komplexe Anionen der verwendeten Metalle. Bevorzugt werden Platin als H₂PtCl₆ oder Pt(NO₃)₂ einge setzt. Als Lösungsmittel für die Metallsalzprecursoren eignen sich Wasser genauso wie organische Lösungsmittel. Besonders geeignet sind niedere Alkohole wie Methanol und Ethanol.

Geeignete Precursoren bei der Verwendung von Edelmetallen als dehydrieraktive Komponente sind auch die entsprechenden Edelmetallsole, die nach einem der bekannten Verfahren, zum Beispiel durch Reduktion eines Metallsalzes in Gegenwart eines Stabilisators wie PVP mit einem Reduktionsmittel hergestellt werden können. Die Herstelltechnik wird in der deutschen Patentanmeldung DE-A-195 00 366 ausführlich behandelt.

Der Katalysator kann im Reaktor fest angeordnet oder z.B. in Form eines Wirbelbettes verwendet werden und eine entsprechende Gestalt haben. Geeignet sind z.B. Formen wie Splitt, Tabletten, Monolithen, Kugeln, oder Extrudate (Stränge, Wagenräder, Sterne, Ringe).

Als Alkali- und Erdalkalimetallprecursor verwendet man in der Regel Verbindungen, die sich durch Calcinieren in die entsprechenden Oxide umwandeln lassen. Geeignet sind zum Beispiel Hydroxide, Carbonate, Oxalate, Acetate oder gemischte Hydroxycarbonate der Alkali- und Erdalkalimetalle.

wird der Mischoxid-Träger zusätzlich oder ausschließlich mit einem Metall der dritten Haupt- oder Nebengruppe dotiert, so sollte man auch in diesem Fall von Verbindungen ausgehen, die sich durch Calcinieren in die entsprechenden Oxide umwandeln lassen. Wird Lanthan verwendet, so sind beispielsweise Lanthan-Oxid-Carbonat, La(OH)₃, La₃(CO₃)₂, La(NO₃)₃ oder Lanthanverbindungen die organische Anionen enthalten, wie La-Acetat, La-Formiat, oder La-Oxalat geeignet.

Die Propan-Dehydrierung wird in der Regel bei Reaktionstemperaturen von 300 bis 800°C, bevorzugt 450 bis 700°C, und einem Druck von 0,1 bis 100 bar, bevorzugt 0,1 bis 40 bar mit einer WHSV (Weight Hourly Space Velocity) von 0,01 bis 100 h⁻¹, bevorzugt 0,1 bis 20 h⁻¹ durchgeführt. Neben dem zu dehydrierenden Kohlenwasserstoff können im Feed Verdünnungsmittel wie beispielsweise CO₂, N₂, Edelgase und/oder Dampf (Wasserdampf), bevorzugt N₂ und/oder Dampf, besonders bevorzugt Dampf zugegen sein.

Ein spezielles Merkmal der erfindungsgemäßen Katalysatoren ist, daß die Katalysatoren bei der Dehydrierung von Kohlenwasserstoffen in Gegenwart von Wasserdampf aktiv sind und die damit verbundenen Vorteile wie Aufhebung der Gleichgewichtslimitierung, Verringerung der Verkokung und Verlängerung der Standzeiten genutzt werden können.

Gegebenenfalls kann zum Kohlenwasserstoff-Feed Wasserstoff zugegeben werden, wobei das Verhältnis von Wasserstoff zu Kohlenwasserstoffstrom in der Regel 0,1:1 bis 100:1, bevorzugt 1:1 bis 20:1 beträgt. Bevorzugt kann die Dehydrierung von Kohlenwasserstoffen mit den erfindungsgemäßen Katalysatoren ohne den Zusatz von Wasserstoff betrieben werden.

Neben der kontinuierlichen Zugabe eines Gases, insbesondere von Dampf (Wasserdampf), gibt es die Möglichkeit, den Katalysator durch Überleiten von Wasserstoff oder Luft von Zeit zu Zeit zu regenerieren. Die Regenerierung selbst findet bei Temperaturen im Bereich 300 bis 900°C, bevorzugt 400 bis 800°C mit einem freien Oxidationsmittel, vorzugsweise mit Luft oder in reduktiver Atmosphäre, vorzugsweise mit Wasserstoff statt. Die Regenerierung kann bei Unterdruck, Normaldruck (Atmosphärendruck) oder Überdruck betrieben werden. Bevorzugt sind Drücke im Bereich 0,5 bis 100 bar.

Für die Dehydrierung mit den erfindungsgemäßen Katalysatoren eignen sich Kohlenwasserstoffe beispielsweise C₂- bis C₁₆-Kohlenwasserstoffe wie Ethan, n-Propan, n-Butan, iso-Butan, n-Pentan, iso-Pentan, n-Hexan, n-Heptan, n-Octan, n-Nonan, n-Decan, n-Undecan, n-Dodecan, n-Tridecan, n-Tetradecan, n-Pentadecan, n-Hexadecan, bevorzugt C₂- bis C₈-Kohlenwasserstoffe wie Ethan, n-Propan, n-Butan, iso-Butan, n-Pentan, iso-Pentan, n-Hexan, n-Heptan, n-Octan, besonders bevorzugt C₂- bis C₄-Kohlenwasserstoffe wie Ethan, n-Propan, n-Butan und iso-Butan, insbesondere Propan und iso-Butan.

Propylen ist ein gefragtes Produkt, insbesondere zur Synthese von Polypropylen oder zur Synthese von funktionalisierten Monomeren und deren Polymerisationsprodukten. Eine Alternative zur Herstellung von Propylen durch Steamcracking von leichtem Naphtha ist die Dehydrierung von Propan.

Isobuten ist ein wichtiges Produkt, insbesondere zur Herstellung von MTBE (Methyl-tert.-butyl-ether). Es wird vor allem in den USA als Kraftstoffadditiv zur Erhöhung der Oktanzahl verwendet. Isobuten läßt sich analog zu Propylen durch Dehydrierung von Isobutan herstellen.

### Beispiele

### Katalysatorherstellung

### Beispiel 1

67,03g ZrO₂ • xSiO₂ **•** xAl₂O₃ (Firma MEL, Bezeichnung XZO 747/03, Splitt 1,6 bis 2 mm) wurden mit einer Lösung von 0,7793g SnCl₂ • 2H₂O und 0,5124 g H₂PtCl₆ • 6H₂O in 400 ml Ethanol übergossen. Die überschüssige Lösung wurde im Rotationsverdampfer innerhalb von 30 Minuten bei einem Druck von 28 mbar im Vakuum entfernt. Die Zusammensetzung wurde 15 h bei 100°C getrocknet und 3 h bei 560°C calciniert. Danach wurde der Katalysator mit einer Lösung von 0,5027g CsNO₃ und 1,7668g KNO₃ in 166 ml übergossen. Die überstehende Lösung wurde innerhalb von 30 Minuten i. Vak. bei einem Druck von 30 mbar entfernt. Der Katalysator wurde 15 h bei 100°C getrocknet und 3 h bei 560°C calciniert.

Der Katalysator hatte eine BET-Oberfläche von 92 m²/g. Durch Quecksilber-Porosimetrie-Messungen erhielt man das Porenvolumen von 0,29 ml/g, die Porenfläche von 67 m²/g und den mittleren Porenradius von 4,9 nm. Bezogen auf das Porenvolumen hatten etwa 31% der Poren einen Durchmesser von weniger als 10 nm und etwa 57% einen Durchmesser zwischen 200 und 4000 nm.

Die Zusammensetzung des Katalysators ist in Tabelle 1 aufgeführt.

### Beispiel 2

186,73 g ZrOCl₂ • 8H₂O wurden in 800 ml Wasser gelöst. Bei Raumtemperatur wurden zu dieser Lösung mit einer Geschwindigkeit von 1 ml/min 347 ml einer 5 M NaOH zugetropft. Nach einer Zeit von etwa 6 h war die Fällung abgeschlossen, der pH-Wert betrug 14. Das Fällgut wurde 15 h bei einer Temperatur von 100°C gealtert. Anschließend wurde die Suspension abfiltriert, mit 3000 ml einer 5%igen NH₄NO₃-Lösung und anschließend mit reinem Wasser gewaschen, bis kein freies Chlor mehr nachweisbar war. Der Feststoff wurde 16 h bei 100°C getrocknet und danach mit einer Aufheizrate von 1°C/min auf 600°C erhitzt und 12 h bei dieser Temperatur calciniert.

110 g eines derart hergestellten ZrO₂-Pulvers wurden mit 3,3 g Walocel in 40 ml Wasser vorgequollen und der Ansatz 2 h geknetet und mit einem Preßdruck von 30 bar zu 3 mm Strängen verarbeitet und anschließend gesplittet.

40 g des zuvor hergestellten Splittes (Siebfraktion 1,6 bis 2 mm) wurden mit einer Lösung aus 0,465 g SnCl₂ • 2H₂O und 0,306 g H₂PtCl₆ • 6H₂O in 245 ml Ethanol übergossen.

Die überschüssige Lösung wurde im Rotationsverdampfer innerhalb von 30 Minuten bei einem Druck von 28 mbar im Vakuum entfernt. Die Zusammensetzung wurde 15 h bei 100°C getrocknet und 3 h bei 560°C calciniert. Danach wurde der Katalysator mit einer Lösung von 0,299 g CsNO₃ und 0,663 g KNO₃ in 105 ml Wasser übergossen. Die überstehende Lösung wurde innerhalb von 30 Minuten i. Vak. bei einem Druck von 30 mbar entfernt. Der Katalysator wurde 15 h bei 100°C getrocknet und 3 h bei 560°C calciniert.

Der Katalysator hatte eine BET-Oberfläche 107 m²/g. Durch Quecksilber-Porosimetrie-Messungen erhielt man das Porenvolumen von 0,46 ml/g, die Porenfläche von 102 m²/g und den mittleren Porenradius von 7,7 nm. Bezogen auf das Porenvolumen hatten etwa 37% der Poren einen Durchmesser von höchstens 10 nm und etwa 40% einen Durchmesser zwischen 200 und 5000 nm.

Die Zusammensetzung des Katalysators ist in Tabelle 1 aufgeführt.

### Beispiel 3

373,46 g ZrOCl₂ • 8H₂O wurden in 3200 ml Wasser gelöst. Bei Raumtemperatur wurden zu dieser Lösung mit einer Geschwindigkeit von 1 ml/min 694 ml einer 5 M NaOH zugetropft. Nach einer Zeit von etwa 6 h war die Fällung abgeschlossen, der pH-Wert betrug 14. Das Fällgut wurde 15 h bei einer Temperatur von 100°C gealtert. Anschließend wurde die Suspension abfiltriert, mit 6000 ml einer 5 %igen NH₄NO₃-Lösung und anschließend mit reinem Wasser gewaschen, bis kein freies C1⁻ mehr nachweisbar war. Der Feststoff wurde 16 h bei 100°C getrocknet. Zu 200 g des derart hergestellten Fällgutes wurden 6 g PVP (Polyvinylpyrrolidon) und 6 g konz. Ameisensäure in 70 ml Wasser gegeben. Der Ansatz wurde 2 h geknetet und bei einem Preßdruck von 20 bar zu 3 mm Strängen verarbeitet und anschließend gesplittet.

40 g des zuvor hergestellten Splittes (Siebfraktion 1,6 bis 2 mm) wurden mit einer Lösung aus 0,639 g SnCl₂ • xH₂O und 0,421 g H₂PtCl₆ **•** 6H₂O in 337 ml Ethanol übergossen. Die überschüssige Lösung wurde im Rotationsverdampfer innerhalb von 30 Minuten bei einem Druck von 28 mbar im Vakuum entfernt. Die Zusammensetzung wurde 15 h bei 100°C getrocknet und 3 h bei 560°C calciniert. Danach wurde der Katalysator mit einer Lösung von 0,411 g CsNO₃ und 0,725 g KNO₃ in 144 ml Wasser übergossen. Die überstehende Lösung wurde innerhalb von 30 Minuten i. Vak. bei einem Druck von 30 mbar entfernt. Der Katalysator wurde 15 h bei 100°C getrocknet und 3 h bei 560°C calciniert.

Der Katalysator hatte eine BET-Oberfläche von 102 m²/g. Durch Quecksilber-Porosimetrie-Messungen erhielt man das Porenvolumen von 0,32 ml/g, die Porenfläche von 101 m²/g und den mittleren Porenradius von 7,8 nm. Bezogen auf das Porenvolumen hatten etwa 50 % der Poren einen Durchmesser von höchstens 10 nm und etwa 25 % einen Durchmesser zwischen 200 und 2000 nm.

Die Zusammensetzung und Ergebnisse des Katalysators sind in Tabelle 1 zusammengestellt.

### Beispiel 4

32 g eines gesplitteten ZrO₂ **•** xSiO₂ Mischoxides der Firma Norton (# 9816590; Siebfraktion 1,6 bis 2 mm) wurden mit einer Lösung aus 0,384 g SnCl₂ • 2H₂O und 0,252 g H₂PtCl₆ • 6H₂O in 196 ml Ethanol übergossen.

Die überschüssige Lösung wurde im Rotationsverdampfer innerhalb von 30 Minuten bei einem Druck von 28 mbar i. Vak. entfernt. Die Zusammensetzung wurde 15 h bei 100°C getrocknet und 3 h bei 560°C calciniert. Danach wurde der Katalysator mit einer Lösung von 0,247 g CsNO₃, 0,435 g KNO₃ und 3,147 g La(NO₃)₃ • 6H₂O in 120 ml H₂O übergossen. Die überstehende Lösung wurde innerhalb von 30 Minuten i. Vak. bei einem Druck von 30 mbar entfernt. Der Katalysator wurde 15 h bei 100°C getrocknet und 3 h bei 560°C calciniert.

Der Katalysator hatte eine BET-Oberfläche 82 m²/g. Durch Quecksilber-Porosimetrie-Messungen erhielt man das Porenvolumen von 0,27 ml/g, die Porenfläche von 65 m²/g und den mittleren Porenradius von 11,7 nm. Bezogen auf das Porenvolumen hatten etwa 58 % der Poren einen Durchmesser von höchstens 20 nm, etwa 18 % der Poren einen Durchmesser von 40 bis 100 nm und etwa 30 % einen Durchmesser von mehr als 40 und weniger als 5000 nm.

Die Zusammensetzung des Katalysators ist in Tabelle 1 aufgeführt.

### Vergleichsbeispiel 1 (Vgl. 1)

Es wurde ein Katalysator nach der Vorschrift in WO-A-94/29021, Beispiel 1 zum Vergleich präpariert (Pt/Sn/Cs/Mg(Al)O).

Die Zusammensetzung des Katalysators ist in Tabelle 1 aufgeführt.

### Vergleichsbeispiel 2 (Vgl. 2)

Der Katalysator wurde analog Vergleichsbeispiel 1 hergestellt.

Die Zusammensetzung des Katalysators ist in Tabelle 1 aufgeführt.

### Katalysatortest

20 ml des zuvor hergestellten Katalysators wurden in einen Rohrreaktor mit einem Innendurchmesser von 22 mm eingebaut. Der Katalysator wurde 30 min bei 580°C mit Wasserstoff versetzt. Danach wurde der Katalysator bei der gleichen Temperatur einem Gemisch aus 80 % Stickstoff und 20 % Luft (Magerluft) ausgesetzt. Nach einer Spülphase von 15 min mit reinem Stickstoff wurde der Katalysator 30 min mit Wasserstoff reduziert. Danach wurde der Katalysator bei einer Reaktionstemperatur von 580°C bzw. 610°C mit 20 Nl/h Propan (99,5 %ig) und H₂O im Molverhältnis Propan/Wasserdampf von 1:1 beaufschlagt. Der Druck betrug 1,5 bar, die GHSV betrug 1000 h⁻¹. Die Reaktionsprodukte wurden gaschromatographisch erfaßt.

Die Ergebnisse mit den Katalysatoren der Beispiele 1 bis 4 und des Vergleichsbeispiels sind in Tabelle 1 zusammengesellt.

**Tabelle 1:**

| Performance der Katalysatoren der Beispiele 1 bis 4 und der Vergleichsbeispiele 1 und 2 in der Propan-Dehydrierung* | | | | | | | | | | | |
|---|---|---|---|---|---|---|---|---|---|---|---|
| | | | | | | | | Umsatz [%] nach | | Selektivität [%] nach | |
| Beispiel Nr./[°C] | Pt [%] | Sn [%] | K [%] | Cs [%] | ZrO₂ [%] | SiO₂ [%] | Al₂O₃ [%] | 1 h | 17 h | 1 h | 17 h |
| 1 /580 | 0,3 | 0,6 | 1,0 | 0,5 | 85,6 | 2,1 | 12,0 | 38 | 36 | 85 | 91 |
| 2 /580 | 0,3 | 0,6 | 0,5 | 0,5 | 98,1 | --- | --- | 41 | 34 | 89 | 85 |
| 3 /580 | 0,3 | 0,6 | 1,0 | 0,5 | 97,6 | --- | --- | 38 | 32 | 92 | 86 |
| 4 /610 | 0,3 | 0,6 | 0,5 | 0,5 | 90,8 | 4,5 | --- | 49 | 45 | 93 | 95 |
| Vgl. 1 /580 | 0,3 | 0,3 | --- | 0,5 | --- | --- | --- | 33 | 29 | 92 | 95 |
| Vgl. 2 /610 | 0,3 | 0,6 | --- | 0,5 | --- | --- | --- | 47 | 38 | 93 | 93 |

| | | | | | | | | | | | |
|---|---|---|---|---|---|---|---|---|---|---|---|
| *) Versuchsbedingungen: 20 ml Katalysator, Splittgröße 1,6 bis 2 mm; 580°C bzw. 610°C; Propan/H₂O 1 : 1 (mol/mol); 20 Nl/h Propan; GHSV = 1000 h⁻¹; 1,5 bar. **) Vergleichskatalysator Pt/Sn/Cs/Mg(Al)O aus WO-A-94/29021 Beispiel 1. | | | | | | | | | | | |

## Patentansprüche

1. Katalysatoren mit bimodaler Porenradienverteilung, bestehend im wesentlichen aus
a) 10 bis 99,9 Gew.-% Zirkondioxid, das zu 50 bis 100 Gew.-% in monokliner Modifikation vorliegt, und
b) 0 bis 60 Gew.-% Aluminiumoxid, Siliciumdioxid und/oder Titandioxid und
c) 0,1 bis 10 Gew.-% mindestens eines aus der ersten oder zweiten Hauptgruppe, der dritten oder achten Nebengruppe des Periodensystems der Elemente und Zinn gewählten Elements,
mit der Maßgabe, daß die Summe der Gewichtsprozente 100 ergibt.

2. Katalysator nach Anspruch 1, **dadurch gekennzeichnet, dass** er im Wesentlichen aus
a) 30 bis 95 Gew.-% Zirkondioxid;
b) 0,5 bis 25 Cew.-% Siliciumdioxid;
c) 0,1 bis 1 Gew.-% Platin; und
d) 0,1 bis 10 Gew.-% mindestens eines aus Kalium, Cäsium, Lanthan und Zinn gewählten Elements
besteht, mit der Maßgabe, dass die Summe der enthaltenen Bestandteile 100 Gew.-% ergibt.

3. Katalysator nach den Ansprüchen 1 bis 2, **dadurch gekennzeichnet, dass** seine BET-Oberfläche im Bereich von 40 bis 300 m²/g liegt.

4. Katalysator nach den Ansprüchen 1 bis 3, **gekennzeichnet dadurch, dass** sein Porenvolumen zwischen 0,25 und 0,5 ml/g liegt.

5. Katalysator nach den Ansprüchen 1 bis 4, **dadurch gekennzeichnet, dass** er 0,1 bis 5 Gew.-% Kalium und/oder Cäsium enthält.

6. Katalysator nach den Ansprüchen 1 bis 5, **dadurch gekennzeichnet, dass** er 0,05 bis 1 Gew.-% Platin und 0,05 bis 2 Gew.-% Zinn enthält.

7. Verfahren zur Herstellung der in den Ansprüchen 1 bis 6 definierten Katalysatoren, **dadurch gekennzeichnet, dass** man der Katalysatorrohmasse 2 bis 30 Gew--% Polyamine, Polyacrylate, Polyalkohole, Polysiloxane, Kohlenhydrate oder deren Gemische, insbesondere Polyvinylpyrrolidon, zu den Katalysatorkomponenten zusetzt und diese oberhalb von 550°C calciniert.

8. Verwendung der in den Ansprüchen 1 bis 6 definierten Katalysatoren zur Dehydrierung von C₂- bis C₁₆-Kohlenwasserstoffen.

9. Verfahren zur Dehydrierung von C₂- bis C₁₆-Kohlenwasserstoffen in Gegenwart eines in den Ansprüchen 1 bis 6 definierten Katalysators.

## Claims

1. A catalyst having a bimodal pore radius distribution and consisting essentially of
a) from 10 to 99,9% by weight of zirconium dioxide of which from 50 to 100% by weight is in the monoclinic modification and
b) from 0 to 60% by weight of aluminum oxide, silicon dioxide and/or titanium dioxide and
c) from 0.1 to 10% by weight of at least one element selected from among main groups one and two and transition groups three and eight of the Periodic Table of the Elements and tin,
with the proviso that the sum of the percentages by weight is 100.

2. A catalyst as claimed in claim 1 which consists essentially of
a) from 30 to 95% by weight of zirconium dioxide;
b) from 0.5 to 25% by weight of silicon dioxide;
c) from 0.1 to 1% by weight of platinum; and
d) from 0.1 to 10% by weight of at least one element selected from among potassium, cesium, lanthanum and tin,
with the proviso that the sum of the constituents present is 100% by weight.

3. A catalyst as claimed in claim 1 or 2 which has a BET surface area in the range from 40 to 300 m²/g.

4. A catalyst as claimed in any of claims 1 to 3 which has a pore volume of from 0.25 to 0.5 ml/g.

5. A catalyst as claimed in any of claims 1 to 4 which contains from 0.1 to 5% by weight of potassium and/or cesium.

6. A catalyst as claimed in any of claims 1 to 5 which contains from 0.05 to 1% by weight of platinum and from 0.05 to 2% by weight of tin.

7. A process for preparing a catalyst as defined in any of claims 1 to 6, wherein from 2 to 30% by weight of polyamines, polyacrylates, polyalcohols, polysiloxanes, carbohydrates or mixtures thereof, in particular polyvinylpyrrolidone, is added to the catalyst components to the raw catalyst compositions [sic] and the mixture is calcined at above 550°C.

8. The use of a catalyst as defined in any of claims 1 to 6 for the dehydrogenation of C₂-C₁₆-hydrocarbons.

9. A process for dehydrogenating C₂-C₁₆-hydrocarbons in the presence of a catalyst as defined in any of claims 1 to 6.

## Revendications

1. Catalyseurs à répartition bimodale des rayons des pores, constitués essentiellement
a) de 10 à 99,9 % en poids de dioxyde de zirconium, qui pour 50 à 100 % en poids se présente sous une forme monoclinique, et
b) de 0 à 60 % en poids d'oxyde d'aluminium, de dioxyde de silicium et/ou de dioxyde de titane, et
c) de 0,1 à 10 % en poids d'au moins un élément choisi parmi le premier ou deuxième groupe principal du système périodique des éléments, son troisième ou huitième groupe secondaire et l'étain,
à la condition que la somme des pour-cents en poids donne 100 %.

2. Catalyseur suivant la revendication 1, **caractérisé en ce qu'**il est essentiellement constitué
a) de 30 à 95 % en poids de dioxyde de zirconium,
b) de 0,5 à 25 % en poids de dioxyde de silicium,
c) de 0,1 à 1 % en poids de platine, et
d) de 0,1 à 10 % en poids d'au moins un élément choisi parmi du potassium, du césium, du lanthane et de l'étain,
à la condition que la somme des éléments contenus donne 100 % en poids.

3. Catalyseur suivant l'une des revendications 1 et 2, **caractérisé en ce que** sa surface spécifique BET est de l'ordre de 40 à 300 m²/g.

4. Catalyseur suivant l'une des revendications 1 à 3, **caractérisé en ce que** son volume poreux est compris entre 0,25 et 0,5 ml/g.

5. Catalyseur suivant l'une des revendications 1 à 4, **caractérisé en ce qu'**il contient 0,1 à 5 % en poids de potassium et/ou de césium.

6. Catalyseur suivant l'une des revendications 1 à 5, **caractérisé en ce qu'**il contient 0,05 à 1 % en poids de platine et 0,05 à 2 % en poids d'étain.

7. Procédé de préparation des catalyseurs définis dans les revendications 1 à 6, **caractérisé en ce que**, dans la masse brute de catalyseur, on ajoute 2 à 30 % en poids de polyamines, de polyacrylates, de polyalcools, de polysiloxanes, d'hydrates de carbone ou de leurs mélanges, en particulier de polyvinylpyrrolidone, par rapport aux composants de catalyseur, et on calcine celle-ci au-dessus de 550°C.

8. Utilisation des catalyseurs définis dans les revendications 1 à 6, pour la déshydrogénation d'hydrocarbures en C₂-C₁₆.

9. Procédé de déshydrogénation d'hydrocarbures en C₂-C₁₆, en présence d'un catalyseur défini dans les revendications 1 à 6.
